# EUROPÄISCHE PATENTSCHRIFT

(11) **EP 1 299 406 B1**
(45) Veröffentlichungstag und Bekanntmachung des Hinweises auf die Patenterteilung: **26.10.2005**
(21) Anmeldenummer: 01953114.4
(22) Anmeldetag: 28.06.2001
(51) Int. Cl.: C07H 15/203, A61K 31/7028, A61P 35/00

(54) **CURCUMIN-DERIVATE MIT GEGENÜBER CURCUMIN VERBESSERTER WASSERLÖSLICHKEIT UND DIESE ENTHALTENDE ARZNEIMITTEL**
CURCUMIN DERIVATIVES WITH IMPROVED WATER SOLUBILITY COMPARED TO CURCUMIN AND MEDICAMENTS CONTAINING THE SAME
DERIVES DE CURCUMINE A SOLUBILITE DANS L'EAU AMELIOREE VIS-A-VIS DE LA CURCUMINE, ET MEDICAMENTS RENFERMANT CES DERIVES

(30) Priorität: 30.06.2000 DE 10031955
(43) Veröffentlichungstag der Anmeldung: 09.04.2003
(73) Patentinhaber: Deutsches Krebsforschungszentrum Stiftung des öffentlichen Rechts, 69120 Heidelberg (DE)
(72) Erfinder: HERGENHAHN, Manfred, 69221 Dossenheim (DE); BERTRAM, Barbara, 69121 Heidelberg (DE); WIESSLER, Manfred, 67227 Frankenthal (DE); SORG, Bernd, L., 60439 Frankfurt (DE)
(74) Vertreter: Schüssler, Andrea
(86) Internationale Anmeldenummer: PCT/DE2001/002337
(87) Internationale Veröffentlichungsnummer: WO 2002/002582

(56) Entgegenhaltungen:
- WO-A-95/18606
- M.-H. PAN ET AL.: "Biotransformation of curcumin through reduction and glucuronidation in mice" DRUG METABOLISM AND DISPOSITION, Bd. 27, Nr. 1, 1999, Seiten 486-494, XP001041393

## Beschreibung

Die vorliegende Erfindung betrifft Curcumin-Derivate mit gegenüber Curcumin verbesserter Wasserlöslichkeit, die dadurch gekennzeichnet sind, daß der Curcumin-Anteil mit einem Saccharid verknüpft ist, sowie diese Derivate enthaltende Arzneimittel. Die erfindungsgemäßen Curcumin-Derivate sind besonders zur Prävention und Behandlung von Krebs, vorzugsweise von EBV-assoziierten Tumoren und Transplantations-assoziierten lymphoproliferativen Erkrankungen, von chronisch-entzündlichen Erkrankungen und von mit einer Retrovirus-Infektion einhergehenden Erkrankung geeignet.

Curcumin (1,7-Bis(4-hydroxy-3-methoxyphenyl)-1,6-heptadien-3,5-dion; Enol-Form) ist der Farbstoff der Gelbwurzgewächse, beispielsweise von Curcuma xanthoriza und Curcuma domestica, und hat sich bisher in Tierversuchen als eine stark wirksame chemopräparative Substanz im Sinne einer tumorhemmenden Wirkung erwiesen, wobei praktisch keine toxischen Wirkungen zu beobachten waren. Curcumin weist darüber hinaus auch eine stark entzündungshemmende Wirkung auf. Schließlich zeigten Curcumin-Analoge in Zellkulturen eine gute inhibitorische Wirkung hinsichtlich der Integrase von HIV (Mazumder et al., J. Med. Chem. 40, S. 3057-3063). Sie können somit die Integration von HIV-DNA nach reverser Transkription in das Wirtsgenom verhindern. Diese Integration ist die Voraussetzung für effiziente Replikation dieser Viren in den Wirtszellen, die bei HIV mit der Krankheitsprognose assoziiert ist. Eine Hemmung der Integrase mittels Curcumin bzw. Curcumin-Analogen kann somit als wichtige therapeutische Maßnahme zur Bekämpfung einer HIV-Infektion angesehen werden. Für Curcumin selbst konnten ähnlich gute Resultate, wie sie bisher im Tiermodell gewonnen wurden, beim Menschen in klinischen Phase I- und Phase-II-Studien nicht erzielt werden, wobei davon ausgegangen werden kann, daß dies darauf beruht, daß der Wirkstoff nicht in ausreichender Konzentration am Wirkort vorliegt. Daten aus einer klinischen Phase-I-Studie in Taiwan zeigen, daß bei einer Verabreichung von 4 g/Tag Curcumin Serumkonzentrationen von lediglich 0,41 µM, bei 6 g/Tag 0,57 µM und bei 8 g/Tag 1,75 µM erreicht wurden, d.h. nur ein geringer Teils des Curcumins wird in die Zirkulation abgegeben und der größere Teil wird ungenutzt ausgeschieden. In dieser Studie wurde Curcumin in Form von Lutschtabletten zu je 100 mg bzw. 1 g Wirkstoff in einer morgendlichen Dosis bis zu 8 g über einen Zeitraum von mehreren Monaten verabreicht. Es ist wahrscheinlich, daß bei den gemessenen geringen Serumkonzentrationen keine Wirkung erreicht werden konnte, da die in der Literatur beschriebenen verschiedenen chemopräventiven Wirkungen von Curcumin in Zellkulturen erst bei Konzentrationen von mindestens 10 µM im Medium auftraten. Schließlich weist die in der Taiwan-Studie eingeschlagene Vorgehensweise noch einen weiteren gravierenden Nachteil auf. Um eine einigermaßen gute Aufnahme des Curcumins im Mund- und Halsbereich überhaupt erzielen zu können, sollten die Patienten die Curcumintabletten im Mund zergehen lassen. Da bei dem in der Taiwan-Studie verwendeten Tablettentyp jede einzelne Tablette mindestens 15 min zur Auflösung brauchte, müßten die Patienten monatelang über längere Zeit des Tages jeweils eine Tablette im Mund zergehen lassen. Es scheint zumindest fragwürdig, ob die Patienten die notwendige Compliance dafür aufbringen.

Pan et al (Drug Metabolism and Disposition, 1999, vol. 27, Seiten 486-494) betrifft Curcumin-Glucuronsäure Derivate die in einer Studie zu Metaboliten von Curcumin in Mäusen verwendet werden. WO 95/18606 betrifft Curcumin Derivate zur Behandlung pathologischer zellproliferativer Erkrankungen.

Somit liegt der vorliegenden Erfindung die Aufgabe zugrunde, Curcumin, Curcumin-Derivate oder Curcumin-Analoge in einer solchen Form bereitzustellen, daß nach Verabreichung höhere Plasmakonzentrationen und somit therapeutisch wirksame Konzentrationen erreicht werden können. Dadurch sollte auch die therapeutisch notwendige Tagesdosis verringert werden können, was zu einer Erhöhung der Bereitschaft des Patienten führen sollte, die Therapie vollständig durchzuführen.

Die Lösung dieses technischen Problems erfolgt durch die in den Patentansprüchen gekennzeichneten Ausführungsformen.

In der vorliegenden Erfindung wurde davon ausgegangen, daß die geringe Aktivität von oral zugeführtem Curcumin beim Menschen eine Folge der geringen Löslichkeit von Curcumin ist, die dann zu den geringen Konzentrationen im Serum führt. Daher wurden Curcumin-Derivate entwickelt, die eine höhere Löslichkeit dadurch aufweisen, daß sie mit Saccharid-Resten verknüpft sind. Diese Curcumin-Derivate können somit beispielsweise als "Prodrugs" durch orale Verabreichung, Injektion oder Infusion zugeführt werden und sollten die Erzielung von Plasmaspiegeln erlauben, bei denen ein therapeutischer Effekt erreichbar ist. Dieses Vorgehen ist auch deshalb vorteilhaft, weil dabei die Diketonstruktur nicht zerstört wird; für die Wirkung von Curcumin ist offenbar diese Struktur erforderlich. Das erfindungsgemäß derivatisierte Curcumin weist nicht nur eine höhere Löslichkeit auf, sondern es kann auch davon ausgegangen werden, daß es deshalb besser von den Zellen aufgenommen wird, weil dafür spezifische Transportsysteme auf der Zelloberfläche zur Verfügung stehen (Veyl et al., PNAS 95, S. 2914-2919 (1998)). Erfindungsgemäße Verbindungen reichern sich deshalb bevorzugt in Zellen, Organen und Geweben an, die Glucosetransporter und/oder verwandte Transporter aufweisen. Die Konjugate sollten sich insbesondere in Leber, Niere, Herz, Thymus, Schilddrüse, Darm und Gehirn sowie in allen Arten von Tumoren anreichern. Durch die Saccharidstrukturen läßt sich auch ein gezieltes Ansteuern (Targeting) von bestimmten Tumorzellen erzielen. So sollten die Curcumin-Derivate vor allem von (prä-)malignen Zellen mit Expression des Glucose-Cotransporters SAAT1 oder ähnlicher Co-Transporter besser aufgenommen werden.

Somit betrifft die vorliegende Erfindung ein Curcumin-Derivat mit gegenüber Curcumin verbesserter Wasserlöslichkeit, dadurch gekennzeichnet, daß der Curcumin-Anteil mit einem Saccharid verknüpft ist wobei das Saccharid keine Glucuronsäure ist. Dies geschieht vorzugsweise über eine glykosidische Bindung.

Der hier verwendete Ausdruck "Curcumin" betrifft sowohl Curcumin als auch Curcumin-ähnliche Verbindungen mit vergleichbarer Aktivität und Analoge davon, die über eine im wesentlichen gleiche Löslichkeit verfügen. Hierzu zählen beispielsweise Dicaffeoylmethan, Rosmarinsäure, Arylamide von Curcumin sowie die Verbindungen NSC 158393 und NSC 117027 (Mazumder et al., J. Med. Chem. 1996, 39: 2472-2481).

Die Verknüpfung des Curcumins bzw. seiner Analoge oder Derivate mit dem Saccharid kann mittels dem Fachmann bekannten Verfahren erfolgen, beispielsweise über die in dem nachstehenden Beispiel beschriebene Königs-Knorr-Reaktion oder über das bekannte Imidat-Verfahren. Zu weiteren geeigneten Verfahren zählt ein Verfahren analog Artico et al. (J. Med. Chem. 41, S. 2984-3960, (1998)), bei dem erfindungsgemäß die Curcumin-Derivate durch Kondensation äquimolarer Mischungen von beispielsweise 4-Hydroxy-3-methoxy-benzaldehyd-4-saccharid und 4-Hydroxy-3-methoxy-benzaldehyd mit Acetylaceton unter Borsäurekatalyse und nachfolgender chromatographischer Abtrennung der gewünschten Mono- oder Disaccharide erhalten werden. Ein alternativer Syntheseweg ist die Herstellung von Saccharidderivaten durch eine "umgekehrte Spaltung" bei der das Reaktionsgleichgewicht der β-Glycosidase-Spaltung durch geeignete Maßnahmen nach links verschoben wird (Menzler et al., 1997, Biotechnology Letters 11 (2), S. 269-272).

Die biologische Wirksamkeit der so erhaltenen Curcumin-Derivate kann anhand der bekannten biologischen Eigenschaften überprüft werden, beispielsweise kann die Curcumin-artige antioxidative Wirkung geprüft werden oder die Hemmung der Transkription zahlreicher viraler und zellulärer Gene, die von AP-1- und NF-kappaB-Stellen enthaltenden Promotoren gesteuert wird.

Der hier verwendete Ausdruck "gegenüber Curcumin verbesserte Wasserlöslichkeit" bezieht sich auf eine solche Wasserlöslichkeit, daß bei oraler Verabreichung, bei Injektion oder bei kontinuierlicher Zuführung, beispielsweise für eine gewünschte Krebs-Prävention (u.U. auch eine Krebs-Chemotherapie), entzündungshemmende oder virushemmende Wirkung, eine ausreichend hohe Konzentration des Wirkstoffs im Serum erreicht werden kann. Vorzugsweise liegt die zu erzielende Serumkonzentration im Bereich von mindestens 5-10 µM. Ganz bevorzugt ist ein Bereich von 10-30 µM.

Der Begriff "Saccharid" umfaßt Saccharide jeglicher Art, insbesondere Mono-, Di-, Oligo- oder Polysaccharide (z.B. mono-, di- tri-, multi-antennäre sowie dendritische Saccharide) in allen stereoisomeren und enantiomeren Formen. Diese können Pentosen oder Hexosen sein. Als Monosaccharide sind insbesondere Glucose, ganz besonders α- und β- D-Glucose, Fructose, Galactose, Mannose, Arabinose, Xylose, Fucose, Rhamnose, Digitoxose und Derivate davon bevorzugt. Als Disaccharide eignen sich insbesondere Saccharose, Maltose, Laktose oder Gentobiose, entweder 1,4- oder 1,6-verknüpft, sowie Derivate davon. Als Saccharide gelten hier auch Inosite und Derivate davon, ganz besonders cis-Inositol, epi-Inositol, allo-Inositol, myo-Inositol, muco-Inositol, chiro-Inositol, neo-Inositol, scyllo-Inositol, Pinpollitol, Streptamin, Quercitol, Chinasäure, Shikimisäure, Conduritol A bzw. B, Validatol und Quebrachitol, z.B. aus Galactinolen, sowohl aus pflanzlichen Quellen, wie Zuckerrüben, als auch aus Milchprodukten, oder durch enzymatische Enantiomerentrennung gewonnene Verbindungen. Ferner sind erfindungsgemäß einsetzbare Saccharide Glycokonjugate. Diese können Konjugate von z.B. Sacchariden mit Peptiden, Lipiden, Säuren (--> Ester), Alkylresten (---> Ether), Heterozyklen oder anderen Kohlenhydraten sein. Ein Beispiel von Glycokonjugaten ist Z1-Z10, ein Gemisch von 10 Glykokonjugaten. Bei den Verbindungen Z1-Z10 handelt es sich um in der Natur vorkommende Glycopeptide, Glycoproteine und Lipopolysaccharide. Derivate der erwähnten Saccharide sind z.B. mit Schutzgruppen, wie Benzyl-, geschützte Saccharide und/oder mit funktionellen Gruppe, wie Aminogruppen, Phosphatgruppen oder Halogenidgruppen, modifizierte Saccharide. Unter Sacchariden werden erfindungsgemäß auch ganze Saccharid-Bibliotheken, wie sie beispielsweise in der deutschen Patentanmeldung DE 196 42 751.7 beschrieben sind, verstanden. Vorstehende Saccharide können natürlich vorkommen oder synthetisch hergestellt sein. Vorzugsweise weist ein erfindungsgemäßes Konjugat nur ein Saccharid auf, aber auch eine Anzahl von 2, 3, 4, 5 und 6 Saccharid-Komponenten ist denkbar. Die Saccharide können dabei gleich oder verschieden voneinander sein.

In einer bevorzugten Ausführungsform der erfindungsgemäßen Konjugate ist ein Saccharid mit der Curcumin-Komponente über einen Linker verbunden. Als Linker eignen sich besonders kurzkettige Diole von 1,2-Diol (z.B. Ethylenglykol) bis 1,6-Hexandiol. Weiter sind Ätherbrücken und Dicarbonsäure-Linker einsetzbar.

Die Derivatisierung wird vorzugsweise so durchgeführt, daß durch spontane oder enzymatisch unterstützte Hydrolyse die Wirksubstanz (Curcumin, Curcumin-ähnliche Verbindungen oder Analoge) in der Zielzelle wieder freigesetzt wird. Dies kann beispielsweise durch extra- oder intrazelluläre β-Glucosidase(n) erfolgen, die ein breites Wirkungsspektrum gegenüber Glykosidderivaten zeigen und in menschlicher Leber und Dünndarm in den relativ höchsten Konzentrationen gegenüber anderen menschlichen Organen vorliegt. Somit sind in einer bevorzugten Ausführungsform der vorliegenden Erfindung die erfindungsgemäßen Curcumin-Derivate dadurch gekennzeichnet, daß die Verknüpfung mit dem Mono-, Oligo- oder Polysaccharid so erfolgt, daß durch spontane, säure- oder enzym-katalysierte Hydrolyse aufgrund der säurelabilen Zuckerbindung an der phenolischen OH-Gruppe in der Zielzelle die Wirksubstanz Curcumin wiederhergestellt wird, wobei die Spaltbarkeit vorher in vitro geprüft werden kann. Besonders bevorzugt sind Curcumin-Derivate, bei denen die Verknüpfung eine O-glykosidische Bindung ist. Vorteilhafterweise erfolgt die Bindung an der 1- oder 4- Position des Saccharids, wobei die 1-Position wegen der besseren Abspaltbarkeit bevorzugt ist.

In der am meisten bevorzugten Ausführungsform ist das erfindungsgemäße Curcumin-Derivat das Curcumin-4-Monoglykosid oder Curcumin-4,4'-Diglykosid bzw. das entsprechende Galactosid.

Schließlich betrifft die vorliegende Erfindung ein Arzneimittel, das ein erfindungsgemäßes Curcumin-Derivat enthält, gegebenenfalls in Kombination mit einem pharmazeutisch verträglichen Träger. Geeignete Träger und die Formulierung derartiger Arzneimittel sind dem Fachmann bekannt. Zu geeigneten Trägern zählen beispielsweise Phosphatgepufferte Kochsalzlösungen, Wasser, Emulsionen, beispielsweise Öl/Wasser-Emulsionen, Netzmittel, sterile Lösungen etc. Das erfindungsgemäße Arzneimittel kann in Form einer Injektionslösung, Tablette, Salbe, Suspension, Emulsion, eines Zäpfchens etc. vorliegen. Es kann auch in Form von Depots (Mikrokapseln, Zinksalze, Liposomen etc.) verabreicht werden. Die Art der Verabreichung des Arzneimittels hängt unter anderen davon ab, in welcher Form der Wirkstoff vorliegt, sie kann oral oder parenteral erfolgen. Zu den Verfahren für die parenterale Verabreichung gehören die topische, intra-arterielle, intra-tumorale (z.B. direkt zu einem Karzinom), intramuskuläre, intramedulläre, intrathekale, intraventrikuläre, intravenöse, intraperitoneale, transdermale oder transmukosale (nasal, vaginal, rektal, sublingual) Verabreichung. Die Verabreichung kann auch durch Mikroinjektion erfolgen. Die geeignete Dosierung wird von dem behandelnden Arzt bestimmt und hängt von verschiedenen Faktoren ab, beispielsweise von dem Alter, dem Geschlecht, dem Gewicht des Patienten, der Art und dem Stadium der Erkrankung, der Art der Verabreichung etc.

Die erfindungsgemäßen Curcumin-Derivate können zusammen mit Glycosidasen (z.B. Cerebrosidase) verabreicht werden, was die Freisetzung des Curcumins unabhängig von im Körper bereits vorhandenen Glycosidasen macht. Die beiden Komponenten können in Liposomen verpackt und verabreicht werden.

Da die tumorhemmende Wirkung von Curcumin im Tierexperiment bereits gezeigt werden konnte, bisher jedoch aufgrund der geringen Löslichkeit von Curcumin beim Menschen deutlich geringer war, kann davon ausgegangen werden, daß mit den erfindungsgemäßen Curcumin-Derivaten aufgrund der deutlich verbesserten Aufnahme und den dadurch erzielten wesentlich erhöhten Plasmaspiegeln eine krebshemmende Wirkung erreicht werden kann. Somit betrifft die vorliegende Erfindung die Verwendung des erfindungsgemäßen Curcumin-Derivats zur Prävention oder Behandlung von Krebs. Eine Hemmung der Epstein-Barr-Virus-Reaktivierung in B-lymphoiden Zellen konnte ebenfalls gezeigt werden. Aufgrund der stark verbesserten Löslichkeit der erfindungsgemäßen Curcumin-Derivate kann davon ausgegangen werden, daß die erfindungsgemäßen Curcumin-Derivate somit auch zur Prävention oder Behandlung von EBV-assoziierten Tumoren, beispielsweise des Nasopharynx-Karzinoms; EBV-haltigen Hodgkin-Lymphomen und -Nicht-Hodgkin-Lymphomen, -T-Zell Lymphomen, -Magencarcinomen, EBV-assoziierte HCV-Hepatitis, EBV-assoziierten Tumoren der weiblichen Brust und Transplantations-assoziierten lymphoproliferativen Erkrankungen (PTLD) geeignet sind. Somit betrifft die vorliegende Erfindung auch die Verwendung der erfindungsgemäßen Curcumin-Derivate zur Prävention oder Behandlung von EBV-assoziierten Tumoren und Transplantations-assoziierten lymphoproliferativen Erkrankungen. Dasselbe gilt auch für andere Viren, wie Hepatitis-B-Viren und humane Papillomviren, bei denen wichtige Gene über Protein-Kinase C-, NF-kappa B, Jun-Kinasen und AP1-Stellen geregelt werden, sowie die mit diesen Viren assoziierten Erkrankungen und Tumoren.

Die vorliegende Erfindung betrifft außerdem die Verwendung der erfindungsgemäßen Curcumin-Derivate zur Behandlung chronisch-entzündlicher Erkrankungen. Hier kommt es auf den antioxidativen Effekt von Curcumin bzw. -Derivaten gegenüber reaktiven Sauerstoff-Spezies aus Entzündungszellen an.

Da bereits gezeigt werden konnte, daß Curcumin-Analoge in Zellkulturen eine hemmende Wirkung auf die Integrase beispielsweise von HIV ausüben, kann davon ausgegangen werden, daß mit den erfindungsgemäßen Curcumin-Derivaten aufgrund der verbesserten Eigenschaften eine wirkungsvolle antivirale Therapie am Menschen erreicht werden kann. Somit betrifft die vorliegende Erfindung schließlich die Verwendung der erfindungsgemäßen Curcumin-Derivate zur Behandlung von mit einer Retrovirus-Infektion, vorzugsweise von mit einer HIV-Infektion einhergehenden Erkrankungen.

### Beschreibung der Figuren:

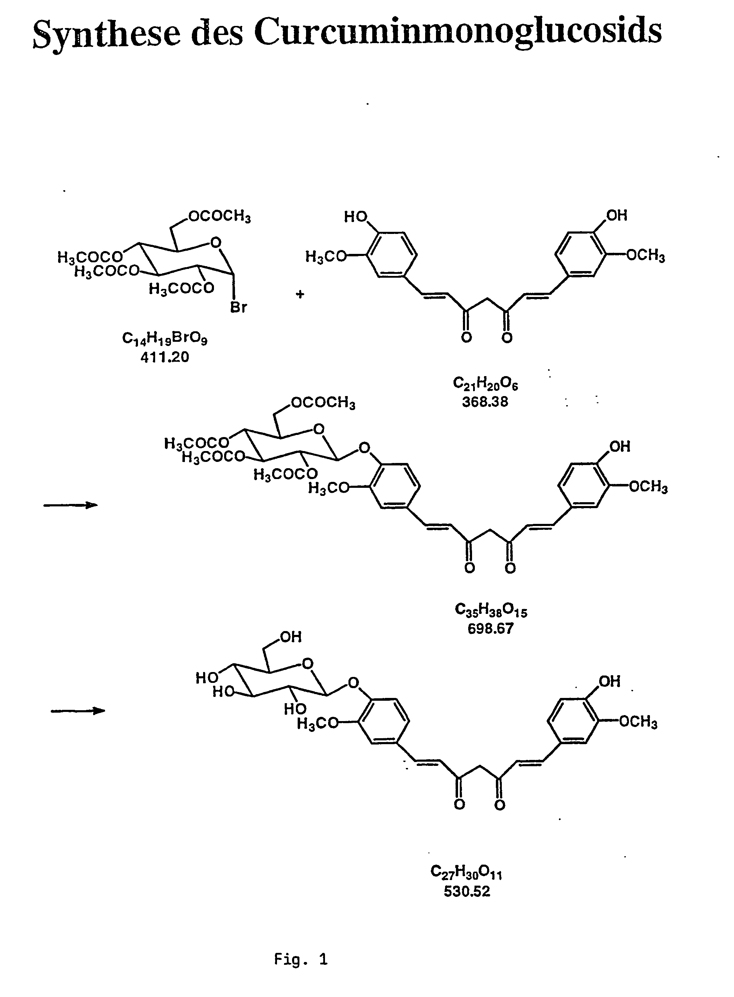

Figur 1: Schematische Darstellung der Synthese des Curcuminmonoglykosids

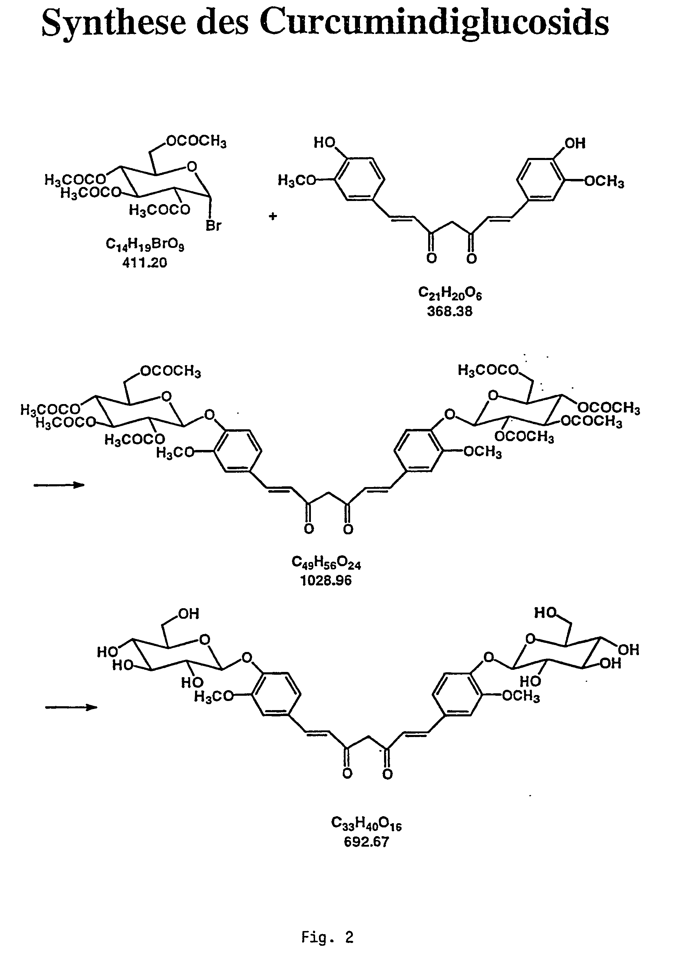

Figur 2: Schematische Darstellung der Synthese des Curcumindiglykosids
Figur 3: Hemmung des "Oxygen Burst" von Granulozyten
Figur 4: Hemmung der EBV-Induktion in Raji-DR-Luc Zellen

Die folgenden Beispiele erläutern die Erfindung.

### Beispiel 1: Herstellung von 8-(2'', 3'', 4'', 6"-Tetra-O-acetyl-β-D-glucopyranosyl)-curcumin (1) und 8-(2'', 3'', 4'',6''-Tetra-O-acetyl-β-D-glucopyranosyl)-8'-(2''', 3''', 4''', 6'''-tetra-O-acetyl-β-D-glucopyranosyl)-curcumin (2)

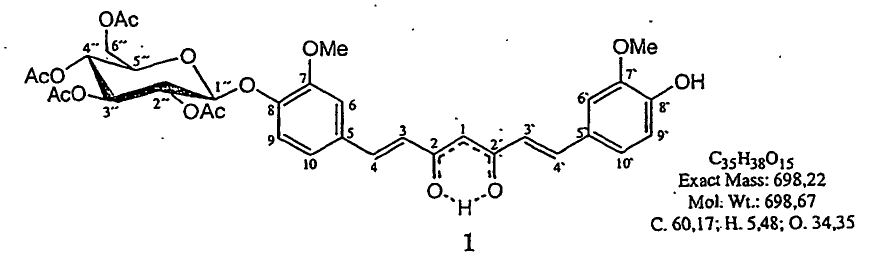

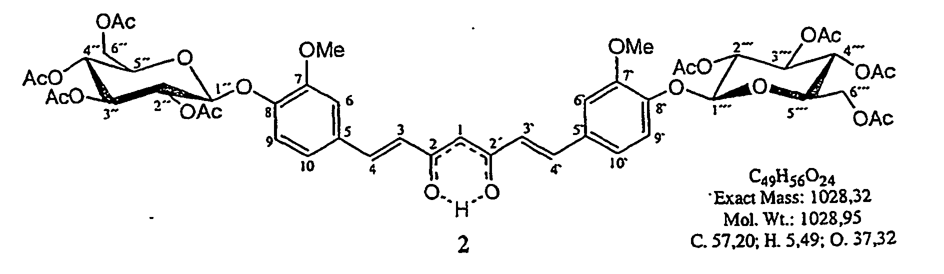

Ein Gemisch aus 0,87 g (3,2 mmol) Benzyltriethylammoniumbromid, 8,25 ml 1,25 M Natronlauge, 16 ml Dichlormethan, 1,64 g (4 mmol) α-D-Acetobromglucose und 2,9 g (8 mmol) Curcumin wurde bei 60° für 12 h intensiv gerührt. Nach dem Abkühlen auf Raumtemperatur wurde die organische Phase abgetrennt, zweimal mit gesättigter wäßriger Natriumchlorid-lösung ausgeschüttelt und zweimal mit Wasser gewaschen. Anschließend wurde die organische Phase über Natriumsulfat getrocknet, abfiltriert und i. Vak. eingeengt. Die Aufreinigung erfolgte durch Säulenchromatographie (Kieselgel, Petrolether/Essigsäureethylester 1,1:1 zur Elution des Monoglucosides, Petrolether/Essigsäureethylester 5:8 zur Elution des Diglucosides)
Ausbeute: 0,395 g (14 % d.Th.): Monoglucosid 0,461 g (11 % d.Th.): Diglucosid 2

### Analytische Daten von 1:

NMR:
   δ_{H} (250 MHz, CDCl₃, 30°C, TMS): 2.04 (s, 6H, COCH₃), 2.08 (s, 6H, COCH₃), 3.80 (ddd, 1H, J_{4",5"} 9.8, J_{5",6a"} 2.5, J_{5",6b"} 4.9, 5"-H), 3.86 (s, 3H, OCH₃), 3.94 (s, 3H, OCH₃), 4.18 (dd, 1H, J_{5",6a"} 2.5, J_{6a",6b"} 12.3, 6a"-H), 4.29 (dd, 1H, J_{5",6b"} 4.9, J_{6a",6b"} 12.3, 6b"-H), 5.01-5.31 (m, 4H, 1"-H, 2"-H, 3"-H, 4"-H), 5.81 (s, 1H, 1-H), 5.92 (bs, 1H, OH), 6.48 (d, 1H, J_{3,4} oder J_{3',4'} 15.9, CHCHCO), 6.51 (d, 1H, J_{3,4} oder J_{3',4'} 15.9. CHCHCO), 6.91-7.14 (m, 6H, 6-H, 9-H, 10-H, 6'-H, 9'-H, 10'-H), 7.57 (d, 1H, J_{3,4} oder J_{3',4'} 15.9, CHCHCO), 7.60 (d, 1H, J_{3,4} oder J_{3',4'} 15.9, CHCHCO)
   δ_{C} (63 MHz, CDCl₃, 30°C): 20.57, 20.60, 20.67 (COCH₃), 55.96, 56,12 (7-OCH₃, 7'-OCH₃) 61.93 (6"-C), 68.38, 71.17, 72.12, 72.54 (2"-C, 3"-C, 4"-C, 5"-C), 100.32, 101.37, 109.71, 111.68, 114.87, 119.64, 121.51, 121.75, 122.96, 123.50 (1-C, 1"-C, 3-C, 3'-C, 6-C, 6'-C, 9-C, 9'-C, 10-C, 10'-C), 127.58, 131.69 (5-C, 5'-C), 139.52, 140.97 (4-C, 4'-C), 146.83, 147.63, 148.00, 150.79 (7-C, 7'-C, 8-C, 8'-C), 169.29, 169.38, 170.23, 170.54 (COCH₃), 182.22, 184.13 (2-C, 2'-C)
Massenspektroskopie:
   pos. ESI-MS (MeOH/CHCl₃ 2:1): *m*/*z* (%): 699.1 [M+H]⁺ (16), 721.1 [M+Na]⁺ (100), 1419.6 [2M+Na]⁺
   neg. ESI-MS (MeOH/CHCl₃ 2:1): *m*/*z* (%): 697.1 [M-H]⁻ (100), 733.0 [M+Cl]⁻ (8)
DC:
   Kieselgel, Petrolether/ Essigsäureethylester (1,1:1) R_{f} = 0,20

### Analytische Daten von 2:

NMR:
   δ_{H} (250 MHz, CDCl₃, 30° C, TMS): 2.04 (s, 12H, COCH₃), 2.08 (s, 12H, COCH₃), 3.81 (ddd, 2H, J_{4",5"} 9.8, J_{5",6a"} 2.5, J_{5",6b"} 5.0, J_{4''',5'''} 9.8, J_{5''',6a'''} 2.5, J_{5''',6b'''} 5.0, 5"-H, 5'''-H), 3.87 (s, 6H, 7-OCH₃, 7'-OCH₃), 4.18 (dd, 2H, J_{5",7a"} 2.5, J_{5",6b"} 5.0, J_{6a",6b"} 12.2, J_{5''',6a'''} 2.5, J_{5''',6b'''} 5.0, J_{6a''',6b'''} 12.2, 6a"-H, 6a'''-H), 4.29 (dd, 2H, J_{5",6b"} 5.0, J_{6a",6b"} 12.2, J_{5''',6b'''} 5.0, J_{6a''',6b'''} 12.2, 6b"-H, 6b'''-H), 5.01-5.31 (m, 8H, 1"-H, 1"'-H, 2"-H, 2'''-H, 3"-H, 3'''-H, 4"-H, 4'''-H), 5,84 (s, 1H, 1-H), 6.52 (d, 2H, J_{3,3'} 16.0, 3-H, 3'-H), 7.08-7.14 (m, 6H, 6-H, 9-H, 10-H, 6'-H, 9'-H, 10'-H), 7.59 (d, 2H J_{4,4'} 16.0, 4-H, 4'-H)
   δ_{C} (63 MHz, CDCl₃, 30°C): 20.54, 20.58, 20.66 (COCH₃), 56.12 (7-OCH₃, 7'-OCH₃), 61,90 (6"-C, 6'''-C), 68.35, 71.15, 72.10, 72.50 (2"-C, 2'''-C, 3"-C, 3'''-C, 4"-C, 4"'-C, 5"-C, 5'''-C), 100.27, 101.56 (1-C, 1"-C, 1'''-C), 111.70, 119.61, 121.59, 123.45 (3-C, 3'-C, 6-C, 6'-C, 9-C, 9'-C, 10-C, 10'-C), 131.55 (5-C, 5'-C), 139.96 (4-C, 4'-C), 147.72, 150.78 (7-C, 7'-C, 8-C, 8'-C), 169.26, 169.36, 170.21, 170.51 (COCH₃), 183.10 (2C, 2'-C)
Massenspektroskopie:
   pos. ESI-MS (MeOH/CHCl₃ 2:1): *m*/*z* (%): 1029.3 [M+H]⁺ (90), 1051.3 [M+Na]⁺ (12), 699.1 [Monoglucosid 1+H]⁺ (80), 2057.7 [2M+H]⁺
DC:
   Kieselgel, Petrolether/ Essigsäureethylester (5:8) R_{f}= 0,22

### Beispiel 2: Herstellung von 8-(β-D-Glucopyranosyl)curcumin (3)

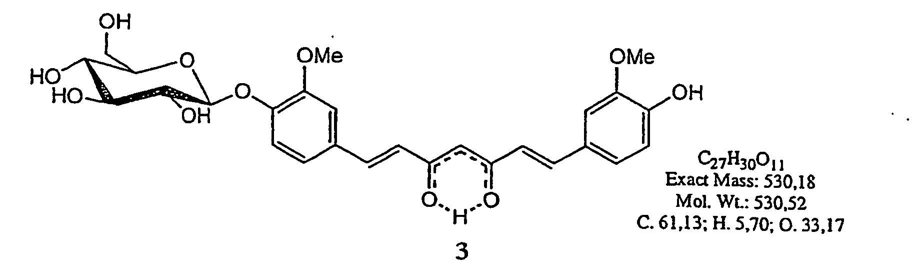

0,375 g (0,51 mmol) acetylgeschütztes Curcuminglucosid 1 wurden in 40 ml Methanol aufgenommen, gerührt, mit 10 ml einer 0,1 M Natriummethanolat-Lösung versetzt und 3 h bei Raumtemperatur weitergerührt. Anschließend wurde mit Ionenaustauscherharz Amberlite H⁺ 50 WX 2 neutralisiert, abfiltriert und i. Vak. eingeengt. Das Reaktionsgemisch wurde säulenchromatographisch aufgetrennt (Kieselgel, Dichlormethan/Methanol 9:1).
Ausbeute: 0,151 g (56 % d.Th.)
DC:
Kieselgel, Dichlormethan/Methanol (9:1) R_{f} = 0,22

### Analytische Daten von 3:

NMR:
   δ_{H} (250 MHz, CD₃OD, 30°C, TMS): 3.42-3.89 (m, 6H, 2"-H, 3"-H, 4"-H, 5"-H, 6a"-H, 6b"-H), 3.89 (s, 6H, 7-OCH₃, 7'-OCH₃), 4.96 (d, 1H, J_{1",2"} 7.4, 1"-H), 5.96 (s, 1H, 1-H), 6.60 (d, 1H, , J_{3,4} oder J_{3',4'} 15.8, CHCHCO), 6,65 (d, 1H, J_{3,4} oder J_{3',4'} 15.8, CHCHCO), 6.79-7.23 (m, 6-H, 6'-H, 9-H, 9'-H, 10-H, 10'-H), 7.54 (d, 1H, J_{3,4} oder J_{3',4'} 15.8, CHCHCO), 7,56 (d, 1H, J_{3,4} oder J_{3',4'} 15,8, CHCHCO)
   δ_{C} (63 MHz, CD₃OD, 30°C): 56.48,56.79 (7-OCH₃, 7'-OCH₃), 62.52 (6"-C), 71.33, 74.85, 77.88,78.31 (2"-C, 3"-C, 4"-C, 5"-C) 102.29 (1"-C) 111.84, 112.51 (6-C, 6'-C), 116.61, 117.52 (9-C, 9'-C), 122.33, 123.44, 123.87, 124.21 (3-C, 3'-C, 10-C, 10'-C), 128.53, 131.42 (5-C, 5'-C), 141.10, 142.51 (4-C, 4'-C), 149.43, 149.88, 150.57, 151.06 (7-C, 7'-C, 8-C, 8'-C), 183.71, 185.65 (2-C, 2'-C)
Massenspektroskopie:
   pos. ESI-MS (MeOH): *m*/*z* (%): 530.6 [M+H]⁺ (4), 552.9 [M+Na]⁺ (100) neg. ESI-MS (MeOH): *m*/*z* (%): 528.9 [M-H]⁻ (100), 564,9 [M+Cl]⁻ (4)
   *m*/*z* (pos. FAB, Nitrobenzylalkohol): gefunden 531,1896 [M+H]⁺, berechnet für C₂₇H₃₁O₁₁ 530,1857

### Beispiel 3: Herstellung von 8,8'-Bis-(β-D-glucopyranosyl) curcumin (4)

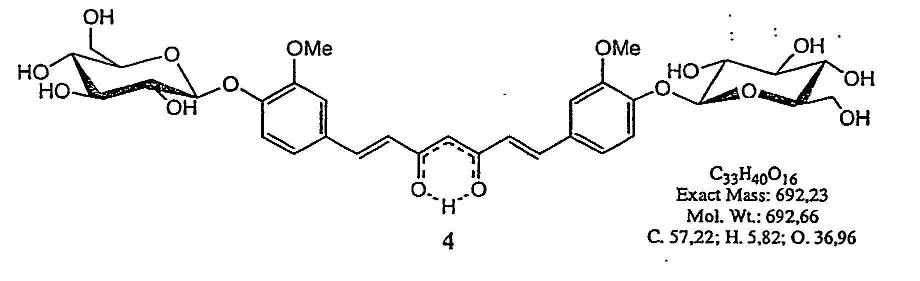

0,381 g (0,37 mmol) acetylgeschütztes Curcuminglucosid 2 wurden in 40 ml Methanol aufgenommen, gerührt, mit 10 ml einer 0,1 M Natriummethanolat-Lösung versetzt und 3 h bei Raumtemperatur weitergerührt. Anschließend wurde mit Ionenaustauscherharz Amberlite H⁺ 50 -wx 2 neutralisiert, abfiltriert und i. Vak. eingeengt. Das Reaktionsgemisch wurde säulenchromatographisch aufgetrennt (Kieselgel, Dichlormethan/Methanol 4:1).
Ausbeute: 0,071 g (28 % d.Th.)

### Analytische Daten von 4:

Massenspektroskopie:
   pos. ESI-MS (MeOH/ H₂O 2:1): *m*/*z* (%):693.1 [M+H]⁺ (4), 715.2 [M+Na]⁺ (72), 1408.2 [2M+Na]⁺ (8)
   neg. ESI-MS (MeOH/H₂O 2:1): *m*/*z* (%): 691.1 [M-H]⁻ (88), 727.1 [M+Cl]⁻ (92)
HR-FAB:
   *m*/*z* (pos. FAB, Glycerin): gefunden 693,2408 [M+H]⁺, berechnet für C₃₃H₄₁O₁₆ 692,2382
DC:
   Kieselgel, Dichlormethan/ Methanol (4:1) R_{f} = 0,20

### Beispiel 4: Wasserlöslichkeit von Curcumin, Curcumin-mono- und -diglucosid

Curcumin und seine zwei Derivate (1) und (2) können mit Hilfe eines HPLC-Systems voneinander getrennt und quantifiziert werden.
HPLC-Bedingungen: Säule Lichrospher-100-RP18-5µ, 125x4 mm Laufmittel/Gradient/Fluss
   Acetonitril/Essigsäure 0.2 bzw. 2 %, 1 ml/min
   Detektion: UV 420 nm

Mit den einzelnen Verbindungen wurde jeweils die zugehörige Eichgerade ermittelt. Zur Bestimmung der Wasserlöslichkeit von Curcumin und seiner beiden Derivate wurden gesättigte Lösungen bei R.T. in Argon-gesättigtem Wasser hergestellt. Der Gehalt von je 20 µl der gesättigten Lösungen wurde mittels HPLC bestimmt.

Zunächst wurden folgende Werte für die Löslichkeit erhalten:

| | |
|---|---|
| Curcumin: | 4 +/-0.3 mg/Liter |
| Curcumin-monoglucosid | 13,2 +/- 0.9 |
| Curcumin-diglucosid | > 10700 |

Daraus ergibt sich eine erhöhte Wasserlöslichkeit der beiden Drivate, vor allem das Diglucosids, bei dem eine gesättigte Lösung mit der verwendeten Menge gar nicht zu erhalten war.

### Beispiel 5: Aufnahme von Curcumin und seinen Derivaten (1) und (2) in Raji-Zellen

Diese Untersuchungen wurden mit Hilfe von Fluoreszenzmikroskopie durchgeführt, da Curcumin und seine beiden Derivate Fluoreszenz zeigen.

Aus Vorversuchen war bekannt, dass Curcumin nach 2-3 Std. maximale Aufnahme in Raji-Zellen zeigt. Raji-Zellen wurden daher jeweils 3 Std. lang mit je 15 µM Curcumin bzw. Derivaten inkubiert.

Aliquots der Zellen in Medium wurden auf Poly-L-lysinbeschichtete Objektträger (zur Anhaftung der Zellen) pipettiert; nach 30 Minuten wurden die Zellen mit einem Deckglas abgedeckt und durch einen geeigneten Filter mit UV-Licht angeregt. Die Fluoreszenz repräsentativer Zellen wurde mit Imageanalyse gemessen; die sehr helle Fluoreszenz des zell-assoziierten Curcumins zeigte, dass vor allem diese Verbindung von den Zellen aufgenommen wird, doch fluoreszierten auch die mit Curcumin-monoglucosid behandelten Zellen um den Faktor 10-100 schwächer, während die mit Curcumin-diglucosid behandelten Zellen gerade noch sichtbar waren.

Dies weist auf eine Aufnahme entweder der unveränderten Verbindungen oder ihres Hydrolyseproduktes Curcumin in B-lymphoide Zellen im Sinne dieser Patentanmeldung hin.

### Beispiel 6: Untersuchungen zur antioxidativen Wirkung von Curcumin und seinen Derivaten (1) und (2)

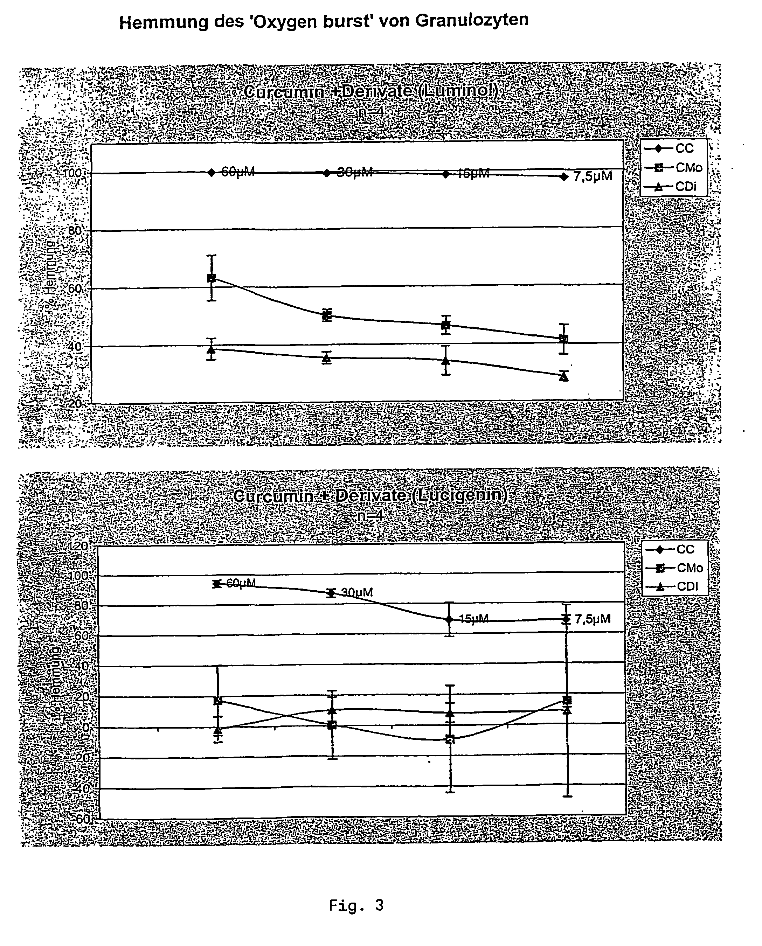

Diese Untersuchungen wurde mit Hilfe der Hemmung des "Oxygen burst" von Granulozyten (PMN) aus menschlichem Blut nach Hergenhahn et al. (1991) J.Cancer Res.Clin.Oncol. 117, 385-395 und Bouvier, Hergenhahn et al. (1993) Carcinogenesis 14, 1573-8, durchgeführt. Die Granulozytenfraktion aus heparinisiertem Blut wird durch Agglutination der Erythrozyten mit 2.5 % Dextran in PBS (30 min bei RT) angereichert, durch Lyse von restlichen Erythrozyten befreit und nach Waschung mit PBS in den Test eingesetzt. In einem Gesamtvolumen von 1 ml werden je 100.000 Granulozyten mit.Luminol bzw. Lucigenin versetzt und im Chemilumineszenz (CL)-Messgerät Biolumat LB 953 (EG&G Berthold, Wildbad) auf 37°C gebracht; die Zellen werden dann mit TPA bei einer Endkonzentration von 100 nM stimuliert. Die CL-Kurven werden über eine Stunde verfolgt; als Mass für die Chemilumineszenz wird das Integral unter der Kurve verwendet. Die Ergebnisse sind in Fig. 3 gezeigt.

Bei Verwendung von Hemmstoffen ergibt sich ein konzentrationsabhängiger Hemmeffekt, der sich als später Anstieg, geringere Amplitude des Maximums und z.T. als früherer Abfall des Peaks erkennbar macht.

Auf diese Weise wurde ein starker antioxidativer Effekt in der Reihe Curcumin » Curcumin-monoglucosid - Curcumin-diglucosid ermittelt.

Daraus kann abgeleitet werden, dass Curcumin und seine Derivate auch antiinflammatorisch aktiv sind, da sie konzentrationsabhängig wesentliche "reaktive Sauerstoff-Spezies" von Entzündungszellen wie Granulozyten und Makrophagen unterdrücken.

### Beispiel 7: Untersuchungen zur Hemmwirkung auf die Reaktivierung von Epstein-Barr-Virus in Raji-zellen

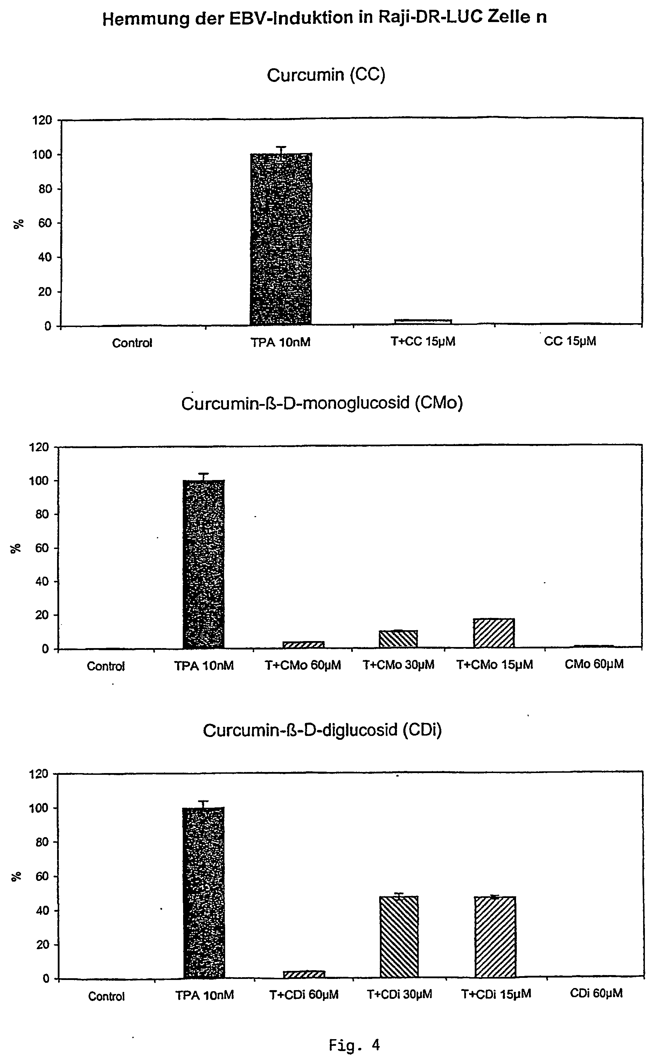

Die gegenwärtigen Untersuchungen wurden mit Raji-DR-LUC-Zellen durchgeführt. Dazu werden 50.000 Raji-DR-Zellen mit 10 nM TPA minus/plus Inhibitor (verschiedene Konzentrationen) in einem Volumen von 100 µl 72 Std. lang im CO2-Inkubator behandelt. Nach Waschen mit PBS werden die Zellen lysiert; die Luciferase-Aktivität wird in Luciferase-Puffer (20 mM Tricine, 8 mM MgSO₄ , 0,1 mM EDTA, 30 mM DTE, pH 7,8) mit Luciferin/CoA ATP als Substrat in einer jeweils gleichen Menge an Zelllysat bestimmt. Die induzierte Luciferase-Aktivität pro µg Protein wird im Vergleich zu einer Eichkurve mit authentischer Luciferase ermittelt. Hemmstoffe der EBV-Induktion führen zu geringerer Induktion von Luciferase im Vergleich mit der TPA-Kontrolle. Das Ergebnis ist in Fig. 4 gezeigt.

Der auf diese Weise ermittelte starke EBV-Hemmeffekt nahm in der Reihe Curcumin > Curcumin-monoglucosid > Curcumin-diglucosid ab.

Curcumin selbst und aus Derivaten freigesetztes Curcumin hemmen die Transaktivation wichtiger Gene, z.B. des Epstein-Barr-Virus, des Hepatitis B Virus, einiger humaner Papillomviren (z.B. HPV 16,18) und weiterer humanpathogener Viren, über sog. AP1-Sequenzen, über NF-kappa B (Familie)-Sequenzen und über bestimmte Signaltransduktionswege, z.B. PKC-, JNK-abhängige Wege; sie besitzen aber vermutlich noch weitere zelluläre Angriffspunkte, wie sich aus ihrer antioxidativen Wirkung ableiten lässt. Daraus kann geschlossen werden, dass sie bei Erreichen genügend hoher Konzentrationen in menschlichen Geweben die Reaktivierung von Epstein-Barr Virus und entzündliche Prozesse hemmen können. Unter denselben Bedingungen kann auch die Synthese und Wirkung wichtiger AP1-regulierter Proteine des Hepatitis B Virus und einiger Human Papillom Viren gehemmt werden.

## Patentansprüche

1. Curcumin-Derivate mit gegenüber Curcumin verbesserter Wasserlöslichkeit, **dadurch gekennzeichnet, daß** der Curcumin-Anteil mit einem oder mehreren Saccharid-Anteilen verknüpft ist, wobei das Saccharid keine Glucuronsäure ist.

2. Curcumin-Derivate nach Anspruch 1, **dadurch gekennzeichnet, daß** die Verknüpfung mit einem Mono-, Oligo- oder Polysaccharid erfolgt.

3. Curcumin-Derivate nach Anspruch 2, wobei die Verknüpfung mit dem Mono-, Oligo- oder Polysaccharid so erfolgt, daß durch spontane oder enzymatische Hydrolyse in der Zielzelle die Wirksubstanz Curcumin freigesetzt wird.

4. Curcumin-Derivate nach Anspruch 1, 2 oder 3, wobei die Verknüpfung eine glykosidische Bindung ist oder mittels eines Linkers erfolgt.

5. Curcumin-Derivate nach einem der Ansprüche 2 bis 4, wobei das Monosaccharid Glucose ist.

6. Curcumin-Derivate nach Anspruch 5, die Curcumin-4-Monoglykosid oder Curcumin-4,4'-Diglykosid sind.

7. Arzneimittel enthaltend Curcumin-Derivate mit gegenüber Curcumin verbesserter Wasserlöslichkeit, wobei der Curcumin-Anteil mit einem oder mehreren Saccharid-Anteilen verknüpft ist.

8. Verwendung eines Curcumin-Derivats mit gegenüber Curcumin verbesserter Wasserlöslichkeit, wobei der Curcumin-Anteil mit einem oder mehreren Saccharid-Anteilen verknüpft ist, zur Herstellung einer pharmazeutischen Zusamensetzung zur Prävention oder Behandlung von Krebs.

9. Verwendung eines Curcumin-Derivats mit gegenüber Curcumin verbesserter Wasserlöslichkeit, wobei der Curcumin-Anteil mit einem oder mehreren Saccharid-Anteilen verknüpft ist, zur Herstellung einer pharmazeutischen Zusamensetzung zur Prävention oder Behandlung von EBV-, HBV- oder HPV-assoziierten Tumoren oder Transplantations-assoziierten lymphoproliferativen Erkrankungen.

10. Verwendung eines Curcumin-Derivats mit gegenüber Curcumin verbesserter Wasserlöslichkeit, wobei der Curcumin-Anteil mit einem oder mehreren Saccharid-Anteilen verknüpft ist, zur Herstellung einer pharmazeutischen Zusamensetzung zur Behandlung chronisch-entzündlicher Erkrankungen.

11. Verwendung eines Curcumin-Derivats mit gegenüber Curcumin verbesserter Wasserlöslichkeit, wobei der Curcumin-Anteil mit einem oder mehreren Saccharid-Anteilen verknüpft ist, zur Herstellung einer pharmazeutischen Zusamensetzung zur Behandlung von mit einer Retrovirus-Infektion einhergehenden Erkrankung.

12. Verwendung nach Anspruch 11, wobei die Retrovirus-Infektion eine HIV-Infektion ist.

## Claims

1. Curcumin derivatives having a water solubility improved as compared to curcumin, **characterized in that** the curcumin part is linked to one or several saccharide portions, the saccharide being no glucuronic acid.

2. The curcumin derivatives according to claim 1, **characterized in that** the linkage takes place by means of a monosaccharide, oligosaccharide or polysaccharide.

3. The curcumin derivatives according to claim 2, wherein the linkage takes place by means of the monosaccharide, oligosaccharide or polysaccharide such that the active substance curcumin is released in the target cell by spontaneous or enzymatic hydrolysis.

4. The curcumin derivatives according to claim 1, 2 or 3, wherein the linkage is a glycosidic bond or effected by means of a linker.

5. The curcumin derivatives according to any of claims 2 to 4, wherein the monosaccharide is glucose.

6. The curcumin derivatives according to claim 5, which are curcumin-4-monoglycoside or curcumin-4,4'-diglycoside.

7. A medicament containing curcumin derivatives having a water solubility improved as compared to curcumin, wherein the curcumin part is linked to one or several saccharide portions.

8. Use of a curcumin derivative having a water solubility improved as compared to curcumin, wherein the curcumin part is linked to one or several saccharide portions, for the preparation of a medicament for the prevention or the treatment of cancer.

9. Use of a curcumin derivative having a water solubility improved as compared to curcumin, wherein the curcumin part is linked to one or several saccharide portions, for the preparation of a medicament for the prevention or the treatment of EBV-, HBV- or HPV-associated tumors or transplantation-associated lymphoproliferative diseases.

10. Use of a curcumin derivative having a water solubility improved as compared to curcumin, wherein the curcumin part is linked to one or several saccharide portions, for the preparation of a medicament for treating chronic-inflammatory diseases.

11. Use of a curcumin derivative having a water solubility improved as compared to curcumin, wherein the curcumin part is linked to one or several saccharide portions, for the preparation of a medicament for treating diseases associated with a retrovirus infection.

12. Use according to claim 11, wherein the retrovirus infection is an HIV infection.

## Revendications

1. Dérivé de curcumine présentant une solubilité dans l'eau supérieure à celle de la curcumine, **caractérisé en ce que** le groupe curcumine est lié à un ou plusieurs groupements saccharide, la saccharide ne consistant pas en acide glucuronique.

2. Dérivé de curcumine selon la revendication 1, **caractérisé en ce que** ladite liaison est réalisée avec un monosaccharide, oligosaccharide ou polysaccharide.

3. Dérivé de curcumine selon la revendication 2, **caractérisé en ce que** la liaison avec un monosaccharide, oligosaccharide ou polysaccharide est réalisée de façon à ce que, dans la cellule cible, la substance active consistant en curcumine soit libérée par hydrolyse spontanée ou enzymatique.

4. Dérivé de curcumine selon la revendication 1, 2 ou 3, **caractérisé en ce que** ladite liaison est une liaison glucosidique ou est réalisée par l'intermédiaire d'un agent de liaison.

5. Dérivé de curcumine selon une quelconque des revendication 2 à 4, **caractérisé en ce que** la monosaccharide consiste en glucose.

6. Dérivé de curcumine selon la revendication 5, **caractérisé en ce que** il consiste en curcumine-4-monoglucoside ou curcumine-4,4'-diglucoside.

7. Médicament contenant un dérivé de curcumine présentant une solubilité dans l'eau supérieure à celle de la curcumine, **caractérisé en ce que** le groupe curcumine est lié à un ou plusieurs groupements saccharide, ladite saccharide ne consistant pas en acide glucuronique

8. Application d'un dérivé de curcumine présentant une solubilité dans l'eau supérieure à celle de la curcumine, dans lequel le groupe curcumine est lié à un ou plusieurs groupements saccharide, pour la fabrication d'une composition pharmaceutique pour la prévention ou le traitement du cancer.

9. Application d'un dérivé de curcumine présentant une solubilité dans l'eau supérieure à celle de la curcumine, dans lequel le groupe curcumine est lié à un ou plusieurs groupements saccharide, pour la fabrication d'une composition pharmaceutique pour la prévention ou le traitement de tumeurs associées à EBV, HBV ou HPV ou le traitement de maladies lymphoprolifératives associées aux transplantations..

10. Application d'un dérivé de curcumine présentant une solubilité dans l'eau supérieure à celle de la curcumine, dans lequel le groupe curcumine est lié à un ou plusieurs groupements saccharide, pour la fabrication d'une composition pharmaceutique pour la prévention ou le traitement de maladies inflammatoires chroniques.

11. Application d'un dérivé de curcumine présentant une solubilité dans l'eau supérieure à celle de la curcumine, dans lequel le groupe curcumine est lié à un ou plusieurs groupements saccharide, pour la fabrication d'une composition pharmaceutique pour la prévention ou le traitement de maladies associées aux infections rétrovirales.

12. Application selon la revendication 11, dans lequel l'infection rétrovirale est une infection VIH.
